# EUROPEAN PATENT APPLICATION

(11) **EP 2 384 744 A1**
(43) Date of publication of application: **09.11.2011**
(21) Application number: 11176711.7
(22) Date of filing: 14.01.2005
(51) Int. Cl.: A61K 9/127

(54) **Lipid-based dispersions useful for drug delivery**

(30) Priority: 14.01.2004 US 536459 P
(62) Divisional of application: 05705671.5
(71) Applicant: Gilead Sciences, Inc., Foster City, CA 94404 (US)
(72) Inventor: Hu, Ning, San Gabriel, California 91775 (US); Jensen, Gerard M., Brea, California 92821 (US); Yang, Stephanie, Temple City, California 91780 (US); Chiang, Su-Ming, Canoga Park, California 91304 (US)
(74) Representative: Reitstötter - Kinzebach

(57) **Abstract**

The invention provides lipid-based dispersion comprising comprising, a) phosphatidyl choline; b) an anionic phospholipid; optionally c) up to 1% cholesterol by weight of total lipids; and optionally d) a therapeutic agent; wherein the mean particle size measured by dynamic light scattering is less than 100 nm. The invention also provides pharmaceutical compositions comprising such a dispersion as well as methods of producing a therapeutic effect in a mammal comprising administering an effective amount of such a dispersion.

## Description

### Background of the Invention

Liposomes are microscopic vesicles made, in part, from phospholipids which form closed, fluid filled spheres when mixed with water. Phospholipid molecules are polar, having a hydrophilic ionizable head, and a hydrophobic tail consisting of long fatty acid chains. When sufficient phospholipid molecules are present in water, the tails spontaneously associate to exclude water. The result is a bilayer membrane in which fatty acid tails converge in the membrane's interior and polar heads point outward toward the aqueous medium. As the liposomes form, water soluble molecules can be incorporated into the aqueous interior, while lipophilic molecules tend to be incorporated into the lipid bilayer. Liposomes may be either multilamellar, onion-like structures, with liquid separating multiple lipid bilayers, or unilamellar, with a single bilayer surrounding an entirely liquid center.

Certain liposomes have been investigated for a variety of purposes in the pharmaceutical field. For example, they have been used to provide targeted delivery, controlled delivery, and sustained release of pharmaceutical agents. See H. T. Balwin and H. R. Six "Liposomes and Immunobiology" (1980) Elsevier North Holland, Inc.; P. T. Kefalides "New Methods of Drug Delivery" (15 June 1998) Annals of Internal Medicine; and D. D. Lasic and D. Papahadjopoulos Eds.

"Medical Applications of Liposomes" (1998) Elsevier Science B.V. They have also been utilized to modify the solubility properties of a variety of therapeutic agents, as well as to modify the toxicity profile of certain agents. In a majority of applications, the emphasis has been on developing liposome/drug combinations that are relatively stable under physiological conditions.

Currently, there are a number of therapeutic agents on the market or in clinical trials that are not used to their full potential because of drug insolubility or carrier vehicle toxicity. These problems are especially true for many lipophilic agents, and for many agents that are administered by injection. Thus, there is currently a need for formulations that can improve the solubility of such therapeutic agents so that they can be utilized to their full potential.

### Summary of the Invention

A lipid-based dispersion has been discovered that is useful for formulating therapeutic agents. Accordingly, the invention provides a lipid-based dispersion comprising, a) phosphatidyl choline; b) an anionic phospholipid; optionally c) up to 1% cholesterol by weight of total lipids; and optionally d) a therapeutic agent; wherein the mean particle size measured by dynamic light scattering is less than 100 nm.

The invention also provides a method for increasing the solubility of a therapeutic agent *in vivo*, (*e.g.* increasing the bio-available amount of the therapeutic agent compared to the amount that is bio-available when the agent is administered in the absence of a lipid-based dispersion of the invention) comprising administering the agent in combination with a lipid-based dispersion of the invention.

The invention also provides a pharmaceutical composition comprising a lipid-based dispersion of the invention.

The invention also provides a unit dosage form comprising a lipid-based dispersion of the invention.

The invention also provides a method for producing a therapeutic effect in an animal comprising administering to the animal an effective amount of a lipid based dispersion of the invention that comprises a therapeutic agent.

The invention also provides a method for modulating the solubility of a therapeutic agent comprising incorporating the agent in a lipid-based dispersion of the invention.

The invention also provides a method for producing an anesthetic or sedative effect in an animal comprising administering to the animal an effective amount of a lipid based dispersion of the invention wherein the therapeutic agent is an anesthetic or a sedative (e.g. propofol).

The invention also provides a method for producing an antineoplastic effect in an animal comprising administering to the animal an effective amount of a lipid based dispersion of the invention wherein the therapeutic agent an antineoplastic agent (e.g. etoposide or paclitaxel).

The invention also provides a method for producing an immunosuppressive effect in an animal comprising administering to the animal an effective amount of a lipid based dispersion of the invention wherein the therapeutic agent is an immunosuppressive agent (e.g. cyclosporine).

The invention also provides a method for treating atherosclerosis, atherosclerotic vulnerable plaque or restenosis, or a combination thereof, in an animal, comprising administering to the animal an effective amount of a lipid based dispersion of the invention wherein the therapeutic agent is an photoreactive agent.

The invention also provides a lipid-based dispersion of the invention for use in medical therapy.

The invention also provides the use of a lipid based dispersion of the invention wherein the therapeutic agent is an anesthetic or a sedative (e.g. propofol) to prepare a medicament useful for producing an anesthetic or sedative effect in a mammal. The invention also provides the use of a lipid based dispersion of the invention wherein the therapeutic agent an antineoplastic agent (e.g. etoposide or paclitaxel), to prepare a medicament useful for producing an antineoplastic effect in a mammal. The invention also provides the use of a lipid based dispersion of the invention wherein the therapeutic agent is an immunosuppressant (e.g. cyclosporine) to prepare a medicament useful for producing an immunosuppressant effect in a mammal.

The invention also provides the use of a lipid based dispersion of the invention wherein the therapeutic agent is a photoreactive agent (e.g. a porphyrin such as gallium deuteroporphyrin dimethyl ester) to prepare a medicament useful for treating atherosclerosis, atherosclerotic vulnerable plaque or restenosis, or a combination thereof, in an animal.

### Brief Description of the Figures

- Figure 1:: shows representative data for cyclosporin from Test A hereinbelow.
- Figure 2:: shows representative data for propofol from Test A hereinbelow
- Figure 3:: shows representative data for etoposide from Test A hereinbelow.

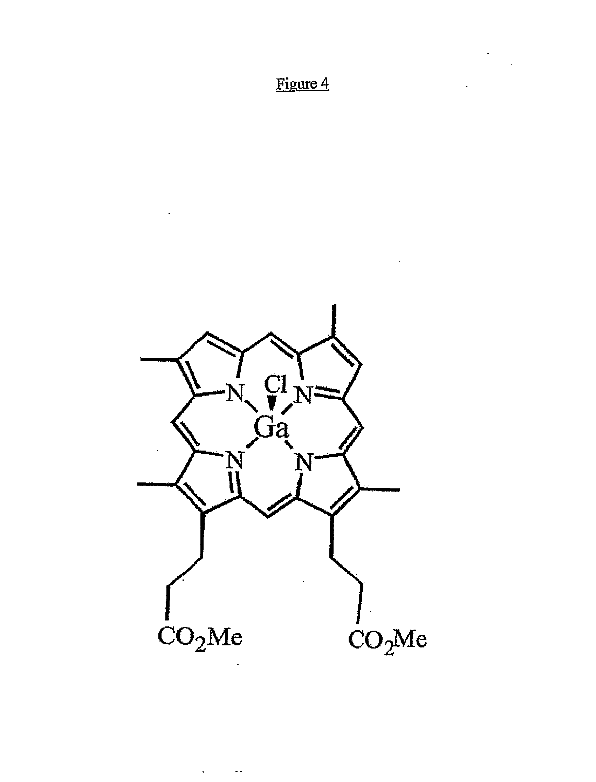

- Figure 4:: depicts the structure of photoreactive agent gallium deuteroporphyrin dimethyl ester.

### Detailed Description

### Phosphatidyl Choline

Typically, the phosphatidyl choline provides the primary packing/entrapment/structural element of the liposome. It provides structurally for the liposome, gives the therapeutic agent a place to go, (both in terms of hydropathy and in terms of flexibility), and establishes a scaffold for the other lipid components. Typically, the phosphatidyl choline comprises mainly C₁₆ or longer fatty-acid chains. Chain length provides for both liposomal structure and membrane width. Additionally, the fatty-acid chains typically have at least one double bond, although this is not a requirement.

As used herein, the term "phosphatidyl choline" includes Soy-PC, Egg-PC, DEPC (dielaidoyl PC), and DOPC (dioleoyl PC), and mixtures thereof, and mixtures of these PC's with other PC's (e.g. mixtures with DSPC, HSPC, or DMPC); the term excludes pure DSPC, HSPC and DMPC.

As used herein, the term "Soy-PC" refers to phosphatidyl choline compositions including a variety of mono-, di-, tri-unsaturated, and saturated fatty acids. Typically, Soy-PC includes palmitic acid present in an amount of about 12% to about 33% by weight; stearic acid present in an amount of about 3% to about 8% by weight; oleic acid present in an amount of about 4% to about 22% by weight; linoleic acid present in an amount of about 55% to about 80% by weight; and linolenic acid present in an amount of about 5% to about 10% by weight.

As used herein, the term "Egg-PC" typically refers to a phosphatidyl choline that comprises palmitic acid in an amount of about 34% by weight; stearic acid in an amount of about 10 % by weight; oleic acid in an amount of about 31% by weight; and linoleic acid in an amount of about 18% by weight.

As used herein, the terms "DEPC" and "DOPC, refer to phosphatidyl choline compositions including C₁₈ fatty acids with one unsaturation and wherein the fatty acid is present in an amount from about 90% to about 100%, preferably, about 100%.

In one embodiment at least about 40% of the fatty-acid chains of the phosphatidyl choline comprise 16 or more carbon atoms.

In another embodiment at least about 50% of the fatty-acid chains of the phosphatidyl choline comprise 16 or more carbon atoms.

In another embodiment at least about 60% of the fatty-acid chains of the phosphatidyl choline comprise 16 or more carbon atoms.

In another embodiment at least about 70% of the fatty-acid chains of the phosphatidyl choline comprise 16 or more carbon atoms.

In another embodiment at least about 80% of the fatty-acid chains of the phosphatidyl choline comprise 16 or more carbon atoms.

In another embodiment at least about 90% of the fatty-acid chains of the phosphatidyl choline comprise 16 or more carbon atoms.

In another embodiment at least about 50% of the fatty-acid chains of the phosphatidyl choline comprise 18 or more carbon atoms.

In another embodiment at least about 60% of the fatty-acid chains of the phosphatidyl choline comprise 18 or more carbon atoms.

In another embodiment at least about 70% of the fatty-acid chains of the phosphatidyl choline comprise 18 or more carbon atoms.

In another embodiment at least about 80% of the fatty-acid chains of the phosphatidyl choline comprise 18 or more carbon atoms.

In another embodiment at least about 90% of the fatty-acid chains of the phosphatidyl choline comprise 18 or more carbon atoms.

In another embodiment at least 50% of the fatty-acid chains of the phosphatidyl choline comprise at least one double bond per chain.

In another embodiment at least 60% of the fatty-acid chains of the phosphatidyl choline comprise at least one double bond per chain.

In another embodiment at least 75% of the fatty-acid chains of the phosphatidyl choline comprise at least one double bond per chain.

In another embodiment at least 50% of the fatty-acid chains of the phosphatidyl choline comprise at least two double bonds per chain.

In another embodiment at least 60% of the fatty-acid chains of the phosphatidyl choline comprise at least two double bonds per chain.

In another embodiment at least 75% of the fatty-acid chains of the phosphatidyl choline comprise at least two double bonds per chain.

In another embodiment the phosphatidyl choline is selected from Soy-PC, Egg-PC, DEPC, and DOPC, and mixtures thereof.

In another embodiment the phosphatidyl choline is Soy-PC.

In another embodiment the phosphatidyl choline is Egg-PC.

### Cholesterol

Cholesterol typically provides a combination of stability and flexibility to liposomal therapeutics. The lipid-based dispersion of the invention typically comprises zero to about 1% cholesterol by weight relative to the total amount of lipids in the dispersion.

In one embodiment, the lipid-based dispersion comprises less than 0.7% cholesterol by weight relative to the amount of total lipid in the dispersion.

In another embodiment the lipid-based dispersion comprises less than 0.5% cholesterol by weight relative to the amount of total lipid in the dispersion.

In another embodiment the lipid-based dispersion comprises less than 0.2% cholesterol by weight relative to the amount of total lipid in the dispersion.

In another embodiment the lipid-based dispersion comprises less than 0.05% cholesterol by weight relative to the amount of total lipid in the dispersion.

In another embodiment the lipid-based dispersion comprises at least about 0.01% cholesterol by weight relative to the amount of total lipid in the dispersion.

In another embodiment the lipid-based dispersion comprises no cholesterol.

### Anionic Phospholipid

The anionic phospholipid typically provides a Coulombic character to the liposomes. This can help stabilize the system upon storage and preventing fusion or aggregation or flocculation; it can also facilitate or enable freeze drying. An anionic surface coating also can contribute to quick biological clearance.

Phospholipids in the phosphatidic acid, phosphatidylglycerol, and phosphatidylserine classes (PA, PG, and PS) are particularly useful in the dispersions of the invention. As with the phosphatidyl choline, the anionic phospholipids typically comprise mainly C₁₆ or larger fatty-acid chains. As used herein, the term "an anionic phospholipid" includes a single anionic phospholipid as well as mixtures of one or more anionic phospholipids.

In one embodiment at least about 60% of the fatty-acid chains of the anionic phospholipid comprise 16 or more carbon atoms.

In another embodiment at least about 70% of the fatty-acid chains of the anionic phospholipid comprise 16 or more carbon atoms.

In another embodiment at least about 80% of the fatty-acid chains of the anionic phospholipid comprise 16 or more carbon atoms.

In another embodiment at least about 90% of the fatty-acid chains of the anionic phospholipid comprise 16 or more carbon atoms.

In another embodiment at least about 60% of the fatty-acid chains of the anionic phospholipid comprise 18 or more carbon atoms.

In another embodiment at least about 70% of the fatty-acid chains of the anionic phospholipid comprise 18 or more carbon atoms.

In another embodiment at least about 80% of the fatty-acid chains of the anionic phospholipid comprise 18 or more carbon atoms.

In another embodiment at least about 90% of the fatty-acid chains of the anionic phospholipid comprise 18 or more carbon atoms.

In another embodiment the anionic phospholipid is selected from Egg-PG (Egg-Phosphatidyglycerol), Soy-PG (Soy-Phosphatidylglycerol), DSPG (Distearoyl Phosphatidyglycerol), DPPG (Dipalmitoyl Phosphatidyglycerol), DEPG (Dielaidoyl Phosphatidyglycerol), DOPG (Dioleoyl Phosphatidyglycerol), DSPA (Distearoyl Phosphatidic Acid), DPPA (Dipalmitoyl Phosphatidic Acid), DEPA (Dielaidoy Phosphatidic Acid), DOPA (Dioleoyl Phosphatidic Acid), DSPS (Distearoyl Phosphatidylserine), DPPS (Dipalmitoyl Phosphatidylserine), DEPS (Dielaidoy Phosphatidylserine), and DOPS (Dioleoyl Phosphatidylserine), and mixtures thereof

In another embodiment the anionic phospholipid is DSPG.

### Liposomes

In certain embodiments of the invention, the lipid-based dispersion comprises liposomes, for example, liposomes having a melting temperature below 35 °C, below 25 °C, or below 15 °C.

### Therapeutic agents

Many highly active and useful pharmaceutical agents suffer from poor solubility. Consequently, the therapeutic use of these pharmaceutical agents is limited. Additionally, some carrier vehicles can be toxic, thereby further limiting the therapeutic agent's use. The lipid-based dispersions of the invention can be used to modify the solubility properties of a therapeutic agent so that the agent can be administered more easily, in a higher dose, or with fewer side-effects. The lipid-based dispersions of the invention are particularly useful for modifying (e.g. improving) the solubility properties of lipophilic therapeutic agents. As used herein, the term therapeutic agent includes diagnostic agents. The term therapeutic agent excludes the compound tacrolimus (FK506).

The lipid-based dispersions of the invention can comprise at least one therapeutic agent including, but not limited to, an analgesic, an anesthetic, an antiacne agent, an antibiotic, an antibacterial, an anticancer, an anticholinergic, an anticoagulant, an antidyskinetic, an antiemetic, an antifibrotic, an antifungal, an antiglaucoma agent, an anti-inflammatory, an antineoplastic, an antiosteoporotic, an antipagetic, an anti-Parkinson's agent, an antipsoriatic, an antipyretic, an antiseptic, an antithrombotic, an antiviral, a calcium regulator, a keratolytic, an immunosuppressant or a sclerosing agent.

Representative therapeutic agents that can be incorporated into a dispersion of the invention include the following agents:
Anesthetics (benzocaine, bupivacaine, chloroprocaine, epinephrine, etidocaine, levobupivacaine, lidocaine, midazolam, oxycondone, phencyclidine, propofol, and ropivacaine);
Antineoplastics (6-diazo-5-oxo-L-norleucine, allopurinol sodium, azaserine, carzinophillin A, denopterin, dolasetron mesylate, edatrexate, eflornithine, erythropoietin, etoposide, fluconazole, melphalan, methotrexate, mycophenolic acid, pamidronate disodium, podophyllinic acid 2-ethylhydrazide, paclitaxel, pteropterin, streptonigrin, Tomudex® (N-((5-(((1,4-Dihydro-2-methyl-4-oxo-6-quinazolinyl)methyl)methylamino)-2-thienyl)carbonyl)-L-glutamic acid), and ubenimex);
Immunosuppressants (azathioprine, basiliximab, bucillamine, cyclosporine, daclizumab, muromonab-CD3, mycophenolic acid, mycophenolate mofetil and other mycophenolate esters, procodazole, Rhₒ(D) immune globulin (human), romurtide, sirolimus, and ubenimex;
Analgesics (acetaminophen, aspirin, hydrocodone, pentosan polysulfate sodium, and phenyl salicylate);
Antiacne Agents (erythromycin, isotretinoin, and tretinoin);
Antibiotics (amikin sulfate, azithromycin, cefazolin, cilastatin, imipenem, minocycline, and penicillin);
Antibacterial Agents (4-sulfanilamidosalicylic acid, acediasulfone, amfenac, amoxicillin, ampicillin, apalcillin, apicycline, aspoxicillin, aztreonam, bambermycin(s), biapenem, carbenicillin, carumonam, cefadroxil, cefamandole, cefatrizine, cefbuperazone, cefclidin, cefdinir, cefditoren, cefepime, cefetamet, cefixime, cefmenoxime, cefminox, cefodizime, cefonicid, cefoperazone, ceforanide, cefotaxime, cefotetan, cefotiam, cefozopran, cefpimizole, cefpiramide, cefpirome, cefprozil, cefroxadine, ceftazidime, cefteram, ceftibuten, ceftriaxone, cefuzonam, cephalexin, cephaloglycin, cephalosporin C, cephradine, ciprofloxacin, clinafloxacin, cyclacillin, enoxacin, epicillin, flomoxef, grepafloxacin, hetacillin, imipenem, lomefloxacin, lymecycline, meropenem, moxalactam, mupirocin, nadifloxacin, norfloxacin, panipenem, pazufloxacin, penicillin N, pipemidic acid, quinacillin, ritipenem, salazosulfadimidine, sparfloxacin, succisulfone, sulfachrysoidine, sulfaloxic acid, teicoplanin, temafloxacin, temocillin, ticarcillin, tigemonam, tosufloxacin, trovafloxacin, and vancomycin);
Anticholinergics (hyoscyamine and oxybutynin);
Anticoagulants (dalteparin, heparin, and warfarin);
Antidyskinetics (amantidine, haloperidol, and tetrabenazine);
Antifibrotics (aprotinin, and desmopressin acetate);
Antifungals (amphotericin B, azaserine, candicidin(s), itraconazole, lucensomycin, natamycin, and nystatin);
Antiglaucoma Agents (brimonidine tartrate, brinzolamide, demecarium bromide, and levobetaxolol);
Anti-inflammatory Agents (glucocorticoids, gold sodium thiomalate, 3-amino-4-hydroxybutyric acid, aceclofenac, alminoprofen, bromfenac, bumadizon, carprofen, diclofenac, diflunisal, enfenamic acid, etodolac, fendosal, flufenamic acid, gentisic acid, meclofenamic acid, mefenamic acid, mesalamine, niflumic acid, olsalazine oxaceprol, S-adenosylmethionine, salsalate, sulfasalazine, and tolfenamic acid)
Antiosteoporotics (raloxifene, sodium fluoride, and teriparatide acetate);
Antipagetics (elcatonin, and tiludonic acid);
Anti-Parkinson's Agents (benztropine mesylate, and biperiden), Antipsoriatics (acitretin, anthralin, lonapalene, tacalcitiol, and tazarotene);
Antipyretics (acetaminosalol, bermoprofen, epirizole, morazone, and salacylamide);
Antiseptics (chlorhexidine gluconate, metronidazole, and sodium sulfacetamide);
Antithrombotics (argatroban, daltroban, iloprost, lamifiban, ozagrel, ridogrel, taprostene, and tirofiban);
Calcium Regulators (calcifediol, calcitonin, ipriflavone, and parathyroid hormone);
Keratolytics (imiquimod, podofilox, and podophyllin); and
Sclerosing Agents (polidocanol, sodium ricinoleate, sodium tetradecyl sulfate, and tribenoside).
Photoreactive Agents (e.g. a porphyrin such as gallium deuteroporphyrin dimethyl ester).
In one embodiment the therapeutic agent is etoposide, propofol, cyclosporin, or paclitaxel.
In one embodiment the therapeutic agent is gallium deuteroporphyrin dimethyl ester.

### Relative Amounts

In one embodiment the lipid-based dispersion comprises from 0.05 to 60 % anionic phospholipid by molar ratio relative to phosphatidyl choline.

In one embodiment the weight ratio of total lipid (phosphatidyl choline + anionic phospholipid) to therapeutic agent is greater than 1:1.

In another embodiment the weight ratio of total lipid (phosphatidyl choline + anionic phospholipid) to therapeutic agent is greater than 5:1.

In another embodiment the weight ratio of total lipid (phosphatidyl choline + anionic phospholipid) to therapeutic agent is greater than 10:1.

In another embodiment the weight ratio of total lipid (phosphatidyl choline + anionic phospholipid) to therapeutic agent is greater than 20:1.

### Formulations

The lipid-based dispersions of the invention can be formulated as pharmaceutical compositions and administered to a mammalian host, such as a human patient in a variety of forms adapted to the chosen route of administration. For example, the lipid-based dispersions of the invention can be formulated to be administered parenterally. Moreover, the lipid-based dispersions can be formulated for subcutaneous, intramuscular, intravenous, or intraperitoneal administration by infusion or injection. These preparations may also contain a preservative to prevent the growth of microorganisms, buffers, or anti-oxidants in suitable amounts.

The lipid-based dispersions of the invention can also be administered orally in combination with a pharmaceutically acceptable vehicle such as an inert diluent or an assimilable edible carrier. They may be enclosed in gelatin capsules or may be incorporated directly with the food of the patient's diet. For oral therapeutic administration, the lipid-based dispersions may be combined with one or more excipients and used in the form of ingestible capsules, elixirs, suspensions, syrups, and the like. Such compositions and preparations will typically contain at least 0.01 % of the therapeutic agent. The percentage of the compositions and preparations may, of course, be varied and may conveniently be between about 0.01 to about 60% of the weight of a given unit dosage form. The amount of therapeutic agent in such therapeutically useful compositions is such that an effective dosage level will be obtained.

For topical administration, the lipid-based dispersions of the invention can be formulated for administration to the skin in combination with a dermatologically acceptable carrier.

Useful dosages of the lipid-based dispersions of the invention can be determined by comparing their *in vitro* activity, and *in vivo* activity in animal models. Methods for the extrapolation of effective dosages in mice, and other animals, to humans are known to the art; for example, see U.S. Pat. No. 4,938,949.

Generally, the concentration of a therapeutic agent in a unit dosage form of the invention will typically be from about 0.01-50% by weight of the composition, preferably from about 0.05-30%, and more preferably 0.1-20% by weight of the composition.

The amount of therapeutic agent required for use in treatment will vary not only with particular agent but also with the route of administration, the nature of the condition being treated and the age and condition of the patient; the amount required will be ultimately at the discretion of the attendant physician or clinician.

The desired amount of a formulation may conveniently be presented in a single dose or as divided doses administered at appropriate intervals, for example, as two, three, four or more sub-doses per day. The sub-dose itself may be further divided, e.g., into a number of discrete loosely spaced administrations.

In one embodiment the lipid based dispersions of the invention have a mean particle size measured by dynamic light scattering of less than about 90 nm, and in another embodiment they have a mean particle size of less than about 80nm.

The ability of a lipid-based dispersion of the invention to successfully deliver a therapeutic agent can be evaluated using a pharmacokinetic study similar to that described in Test A below.

### Test A.

Male Sprauge-Dawley rats were dosed intravenously with a liposomal formulation of either cyclosporin, propofol, or etoposide. For comparison rats were also dosed with a commercially available formulation of cyclosporine (Sandimmune Injection from Novartis lot # 135), propofol (Diprivan (Propofol) from Astra Zeneca Pharmaceuticals), or etoposide (Etoposide for Injection from GensiaSicor Pharmaceuticals). Animals were dosed at 5 mg/kg for cyclosporin, 8mg/kg for propofol and 10mg/kg for etoposide. Blood samples were drawn prior to dosing and at 5, 30, 60 120, 240, 360, 480, 720 1440 and 2880 minutes after dosing. Samples were then analyzed for drug levels by HPLC.

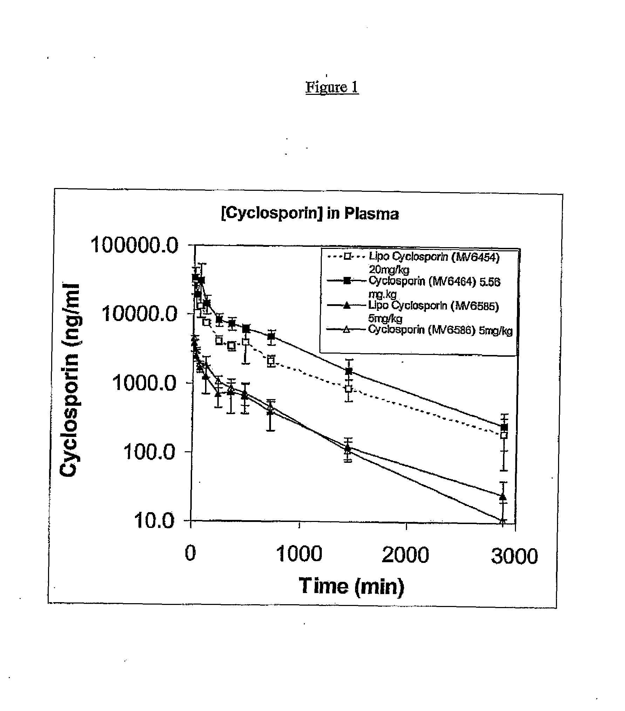

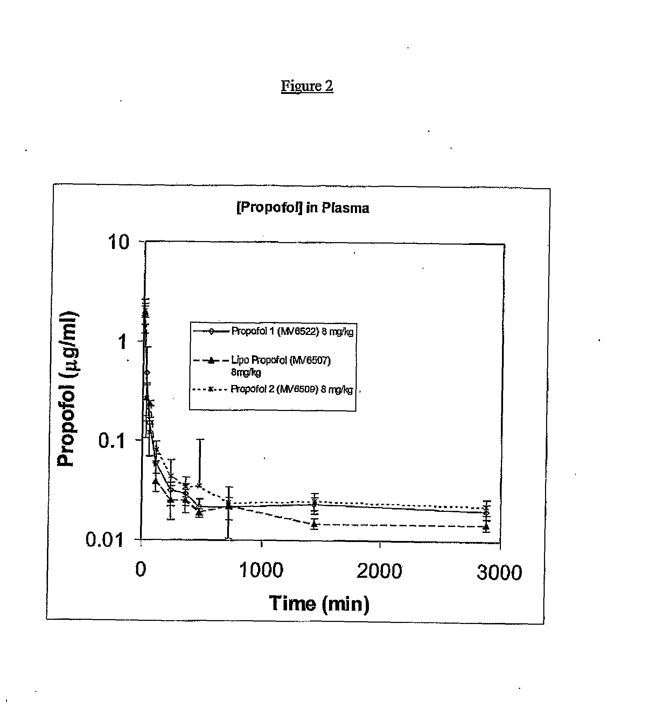

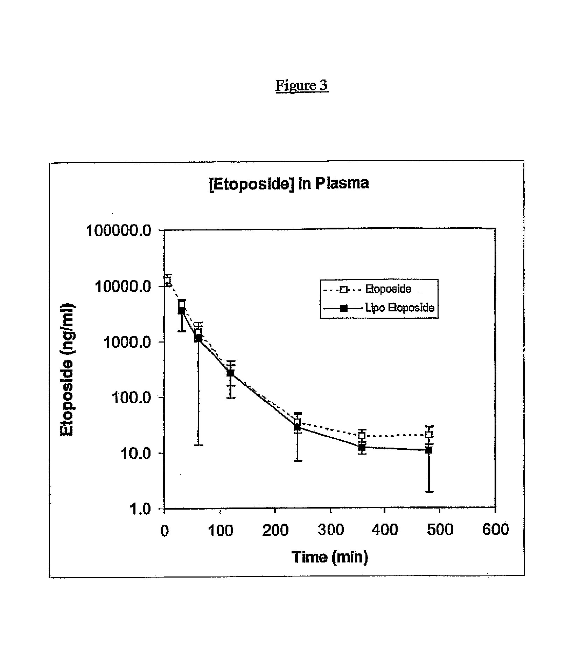

Representative data for cyclosporine, propofol, and etoposide from Test A is shown in Figures 1-3. This data demonstrates that the liposomal-cyclosporin (at 5mg/kg), liposomal-etoposide and liposomal-propofol of the invention appear equivalent to the commercial formulation in Cmax and in AUC as well as clearance of the drug.

The invention will now be illustrated by the following non-limiting Examples.

### EXAMPLES

### Example 1. Lipid-based Dispersion of Etoposide

Soy-PC, DSPG and etoposide were dissolved in a 1:1 (v:v) mixture of methanol and chloroform at a molar ratio of Soy-PC:DSPG of 1:0.2 and a weight ratio of (Soy-PC + DSPG):etoposide of 20:1. Once all components were dissolved, solvents were removed by evaporation under continuous nitrogen flow. Residual solvent was removed by storing the tube containing the material in a desiccator under vacuum for not less than 48 hours. The films were then hydrated in 9% sucrose at desired drug concentrations and sonicated to form liposomes. The resulting solution was filtered through a 0.2-micron filter and evaluated.

### Example 2. Lipid-based Dispersion of Cyclosporin

Soy-PC, DSPG and cyclosporin were dissolved in a 1:1 (v:v) mixture of methanol and chloroform at a molar ratio of Soy-PC:DSPG of 2:0.5 and a weight ratio of (Soy-PC + DSPG):cyclosporin of 20:1. Once all components were dissolved, solvents were removed by evaporation under continuous nitrogen flow. Residual solvent was removed by storing the tube containing the material in a desiccator under vacuum for not less than 48 hours. The films were then hydrated in 9% sucrose at desired drug concentrations and sonicated to form liposomes. The resulting solution was filtered through a 0.2-micron filter and evaluated.

### Example 3. Lipid-based Dispersion of Propofol

Soy-PC, DSPG and propofol were dissolved in a 1:1 (v:v) mixture of methanol and chloroform at a molar ratio of Soy-PC:DSPG of 1:0.4 and a weight ratio of (Soy-PC + DSPG):propofol of 10:1. Once all components were dissolved, solvents were removed by evaporation under continuous nitrogen flow. Residual solvent was removed by storing the tube containing the material in a desiccator under vacuum for not less than 48 hours. The films were then hydrated in 9% sucrose at desired drug concentrations and sonicated to form liposomes. The resulting solution was filtered through a 0.2-micron filter and evaluated.

### Example 4. Lipid-based Dispersion of Gallium Deuteroporphyrin Dimethyl Ester

Soy-PC, DSPG and gallium deuteroporphyrin dimethyl ester were dissolved in chloroform at a molar ratio of Soy-PC: DSPG of 1:0.3 and a weight ratio of (Soy-PC + DSPG): gallium deuteroporphyrin dimethyl ester of 20:1. Once all components were dissolved, solvents were removed by evaporation under continuous nitrogen flow. Residual solvent was removed by storing the tube containing the material in a desiccator under vacuum for not less than 48 hours. The films were then hydrated in 9% sucrose at desired drug concentrations and sonicated to form liposomes. The resulting solution was filtered through a 0.2-micron filter and evaluated.

### Example 5. Lipid-based Dispersion of Gallium Deuteroporphyrin Dimethyl Ester

Soy-PC, DSPG and gallium deuteroporphyrin dimethyl ester were dissolved in chloroform at a molar ratio of Soy-PC: DSPG of 1:0.1 and also at 1:0.4 and a weight ratio of (Soy-PC + DSPG): gallium deuteroporphyrin dimethyl ester of 20:1. Once all components were dissolved, solvents were removed by evaporation under continuous nitrogen flow. Residual solvent was removed by storing the tube containing the material in a desiccator under vacuum for not less than 48 hours. The films were then hydrated in 9% sucrose at desired drug concentrations and sonicated to form liposomes. The resulting solution was filtered through a 0.2-micron filter and evaluated. Testing for plasma precipitation (rabbit plasma) and for blood stability (rabbit blood with visual analysis for hemolysis) indicated no plasma precipitation and very low to no levels of hemolysis.

### Example 6. The following illustrate representative pharmaceutical dosage forms, containing a lipid-based dispersion of the invention, for therapeutic or prophylactic use in humans.

| (i) Injection 1 (1 mg/ml) | mg/ml |
|---|---|
| 'Therapeutic Agent' | 1.0 |
| Phosphatidyl choline | 28.3 |
| Anionic Phospholipid | 11.7 |
| Sucrose | 90 |
| 0.1 N Sodium hydroxide solution | |
| (pH adjustment to 7.0-7.5) | q.s. |
| Water for injection | q.s. ad 1 mL |

| (ii) Injection 2 (10 mg/ml) | mg/ml |
|---|---|
| `Therapeutic Agent' | 10 |
| Phosphatidyl choline | 58 |
| Anionic Phospholipid | 12 |
| 0.1 N Sodium hydroxide solution | |
| (pH adjustment to 7.0-7.5) | q.s. |
| sucrose | 90 |
| Water for injection | q.s. ad 1 mL |

The above formulations may be obtained by conventional procedures well known in the pharmaceutical art.

All publications, patents, and patent documents are incorporated by reference herein, as though individually incorporated by reference. The invention has been described with reference to various specific and preferred embodiments and techniques. However, it should be understood that many variations and modifications may be made while remaining within the spirit and scope of the invention.

### Embodiments of the invention

1. A lipid-based dispersion comprising, a) phosphatidyl choline; b) an anionic phospholipid; optionally c) up to 1%cholesterol by weight of total lipids; and optionally d) a therapeutic agent; wherein the mean particle size measured by dynamic light scattering is less than 100 nm.
2. The lipid-based dispersion of embodiment 1 wherein at least about 60% of the fatty-acid chains of the phosphatidyl choline comprise 16 or more carbon atoms.
3. The lipid-based dispersion of embodiment 1 wherein at least about 70% of the fatty-acid chains of the phosphatidyl choline comprise 16 or more carbon atoms.
4. The lipid-based dispersion of embodiment 1 wherein at least about 80% of the fatty-acid chains of the phosphatidyl choline comprise 16 or more carbon atoms.
5. The lipid-based dispersion of embodiment 1 wherein at least about 90% of the fatty-acid chains of the phosphatidyl choline comprise 16 or more carbon atoms.
6. The lipid-based dispersion of embodiment 1 wherein at least about 60% of the fatty-acid chains of the phosphatidyl choline comprise 18 or more carbon atoms.
7. The lipid-based dispersion of embodiment 1 wherein at least about 70% of the fatty-acid chains of the phosphatidyl choline comprise 18 or more carbon atoms.
8. The lipid-based dispersion of embodiment 1 wherein at least about 80% of the fatty-acid chains of the phosphatidyl choline comprise 18 or more carbon atoms.
9. The lipid-based dispersion of embodiment 1 wherein at least about 90% of the fatty-acid chains of the phosphatidyl choline comprise 18 or more carbon atoms.
10. The lipid-based dispersion of anyone of embodiments 1-9 wherein at least 50% of the fatty-acid chains of the phosphatidyl choline comprise at least one double bond.
11. The lipid-based dispersion of anyone of embodiments 1-9 wherein at least 60% of the fatty-acid chains of the phosphatidyl choline comprise at least one double bond.
12. The lipid-based dispersion of anyone of embodiments 1-9 wherein at least 75% of the fatty-acid chains of the phosphatidyl choline comprise at least one double bond.
13. The lipid-based dispersion of embodiment 1 wherein the phosphatidyl choline is selected from Soy-PC, Egg-PC, DEPC, and DOPC.
14. The lipid-based dispersion of embodiment 1 wherein the phosphatidyl choline is Soy-PC.
15. The lipid-based dispersion of embodiment 1 wherein the phosphatidyl choline is Egg-PC.
16. The lipid-based dispersion of anyone of embodiments 1-15 that comprises less than 0.5% cholesterol.
17. The lipid-based dispersion of anyone of embodiments 1-15 that comprises less than 0.05% cholesterol.
18. The lipid-based dispersion of anyone of embodiments 1-15 that comprises no cholesterol.
19. The lipid-based dispersion of anyone of embodiments 1-18 wherein at least about 60% of the fatty-acid chains of the anionic phospholipid comprise 14 or more carbon atoms.
20. The lipid-based dispersion of anyone of embodiments 1-18 wherein at least about 70% of the fatty-acid chains of the anionic phospholipid comprise 16 or more carbon atoms.
21. The lipid-based dispersion of anyone of embodiments 1-18 wherein at least about 80% of the fatty-acid chains of the anionic phospholipid comprise 16 or more carbon atoms.
22. The lipid-based dispersion of anyone of embodiments 1-18 wherein at least about 90% of the fatty-acid chains of the anionic phospholipid comprise 16 or more carbon atoms.
23. The lipid-based dispersion of anyone of embodiments 1-18 wherein at least about 60% of the fatty-acid chains of the anionic phospholipid comprise 18 or more carbon atoms.
24. The lipid-based dispersion of any one of embodiments 1-18 wherein at least about 70% of the fatty-acid chains of the anionic phospholipid comprise 18 or more carbon atoms.
25. The lipid-based dispersion of anyone of embodiments 1-18 wherein at least about 80% of the fatty-acid chains of the anionic phospholipid comprise 18 or more carbon atoms.
26. The lipid-based dispersion of anyone of embodiments 1-18 wherein at least about 90% of the fatty-acid chains of the anionic phospholipid comprise 18 or more carbon atoms.
27. The lipid-based dispersion of anyone of embodiments 1-18 wherein the anionic phospholipid is selected from Egg-PG, Soy-PG, DSPG, DPPG, DEPG, DOPG, DSPA, DPPA, DEPA, DOPA, DSPS, DPPS, DEPS, and DOPS, and mixtures thereof
28. The lipid-based dispersion of anyone of embodiments 1-18 wherein the anionic phospholipid is DSPG.
29. The lipid-based dispersion of anyone of embodiments 1-28 which comprises a therapeutic agent.
30. The lipid-based dispersion of embodiment 29 wherein the therapeutic agent is an analgesic, anesthetic, antiacne agent, antibiotic, antibacterial, anticholinergic, anticoagulant, antidyskinetic, antifibrotic, antifungal, antiglaucoma agents, anti-inflammatory, antineoplastic, antiosteoporotic, antipagetic, anti-Parkinson's agent, antipsoriatic, antipyretic, antiseptic, antithrombotic, calcium regulator, keratolytic, an immunosuppressant, a photoreactive agent, or a sclerosing agent.
31. The lipid-based dispersion of embodiment 29 wherein the therapeutic agent is benzocaine, bupivacaine, chloroprocaine, epinephrine, etidocaine, levobupivacaine, lidocaine, midazolam, oxycondone, phencyclidine, propofol, ropivacaine, 6-diazo-5-oxo-L-norleucine, allopurinol sodium, azaserine, carzinophillin A, denopterin, dolasetron mesylate, edatrexate, eflornithine, erythropoietin, etoposide, fluconazole, melphalan, methotrexate, mycophenolic acid, pamidronate disodium, podophyllinic acid 2-ethylhydrazide, paclitaxel, pteropterin, streptonigrin, Tomudex® (N-((5-(((1,4-Dihydro-2-methyl-4-oxo-6-quinazolinyl)methyl)methylamino)-2-thienyl)carbonyl)-L-glutamic acid), ubenimex, azathioprine, basiliximab, bucillamine, cyclosporine, daclizumab, muromonab-CD3, mycophenolic acid, mycophenolate mofetil, procodazole, Rho(D) immune globulin (human), romurtide, sirolimus, ubenimex, acetaminophen, aspirin, hydrocodone, pentosan polysulfate sodium, phenyl salicylate, erythromycin, isotretinoin, tretinoin, amikin sulfate, azithromycin, cefazolin, cilastatin, imipenem, minocycline, penicillin, 4-sulfanilamidosalicylic acid, acediasulfone, amfenac, amoxicillin, ampicillin, apalcillin, apicycline, aspoxicillin, aztreonam, bambermycin(s), biapenem, carbenicillin, carumonam, cefadroxil, cefamandole, cefatrizine, cefbuperazone, cefclidin, cefdinir, cefditoren, cefepime, cefetamet, cefixime, cefmenoxime, cefminox, cefodizime, cefonicid, cefoperazone, ceforanide, cefotaxime, cefotetan, cefotiam, cefozopran, cefpimizole, cefpiramide, cefpirome, cefprozil, cefroxadine, ceftazidime, cefteram, ceftibuten, ceftriaxone, cefuzonam, cephalexin, cephaloglycin, cephalosporin C, cephradine, ciprofloxacin, clinafloxacin, cyclacillin, enoxacin, epicillin, flomoxef, grepafloxacin, hetacillin, imipenem, lomefloxacin, lymecycline, meropenem, moxalactam, mupirocin, nadifloxacin, norfloxacin, panipenem, pazufloxacin, penicillin N, pipemidic acid, quinacillin, ritipenem, salazosulfadimidine, sparfloxacin, succisulfone, sulfachrysoidine, sulfaloxic acid, teicoplanin, temafloxacin, temocillin, ticarcillin, tigemonam, tosufloxacin, trovafloxacin, vancomycin, hyoscyamine, oxybutynin, dalteparin, heparin, warfarin, amantidine, haloperidol, tetrabenazine, aprotinin, desmopressin acetate, amphotericin B, azaserine, candicidin(s), itraconazole, lucensomycin, natamycin, nystatin, brimonidinetartrate, brinzolamide, demecarium bromide, levobetaxolol, a glucocorticoid, gold sodium thiomalate, 3-amino-4-hydroxybutyric acid, aceclofenac, alminoprofen, bromfenac, bumadizon, carprofen, diclofenac, diflunisal, enfenamic acid, etodolac, fendosal, flufenamic acid, gentisic acid, meclofenamic acid, mefenamic acid, mesalamine, niflumic acid, .olsalazine oxaceprol, S-adenosylmethionine, salsalate, sulfasalazine, tolfenamic acid, raloxifene, sodium fluoride, and teriparatide acetate, elcatonin, tiludonic acid, benztropine mesylate, biperiden, acitretin, anthralin, lonapalene, tacalcitiol, tazarotene, acetaminosalol, bermoprofen, epirizole, morazone, salacylamide, chlorhexidine gluconate, metronidazole, sodium sulfacetamide, argatroban, daltroban, iloprost, lamifiban, ozagrel, ridogrel, taprostene, tirofiban, calcifediol, calcitonin, ipriflavone, parathyroid hormone, imiquimod, podofilox, podophyllin, polidocanol, sodium ricinoleate, sodium tetradecyl sulfate, or tribenoside.
32. The lipid-based dispersion of embodiment 29 wherein the therapeutic agent is etoposide, propofol, cyclosporin, or paclitaxel.
33. The lipid-based dispersion of embodiment 29 wherein the therapeutic agent is gallium deuteroporphyrin dimethyl ester.
34. The lipid-based dispersion of anyone of embodiments 1-33 that comprises liposomes.
35. The lipid-based dispersion of embodiment 34 wherein the liposomes have a melting temperature below 35°.
36. The lipid-based dispersion of embodiment 34 wherein the liposomes have a melting temperature below 25°.
37. The lipid-based dispersion of embodiment 34 wherein the liposomes have a melting temperature below 15°.
38. The lipid-based dispersion of anyone of embodiments 1-37 which comprises from 0.05 to 60 % anionic phospholipid by mole relative to phosphatidyl choline.
39. The lipid-based dispersion of anyone of embodiments 1-38 wherein the weight ratio of total lipid (phosphatidyl choline +anionic phospholipid) to therapeutic agent is greater than 1: 1.
40. The lipid-based dispersion of any one of embodiments 1-38 wherein the weight ratio of total lipid (phosphatidyl choline +anionic phospholipid) to therapeutic agent is greater than 5:1.
41. The lipid-based dispersion of anyone of embodiments 1-38 wherein the weight ratio of total lipid (phosphatidyl choline +anionic phospholipid) to therapeutic agent is greater than 10: 1.
42. The lipid-based dispersion of anyone of embodiments 1-38 wherein the weight ratio of total lipid (phosphatidyl choline +anionic phospholipid) to therapeutic agent is greater than 20: 1.
43. A unit dosage form comprising a lipid-based dispersion of anyone of embodiments 1-42.
44. The unit dosage form of embodiment 43, which is formulated for parenteral administration.
45. The unit dosage form of embodiment 43, which is formulated for oral administration.
46. A method for modulating the solubility of a therapeutic agent comprising incorporating the agent in a lipid-based dispersion as described in anyone of embodiments 1-42.
47. A method for producing an anesthetic or sedative effect in an animal comprising administering to the animal an effective amount of a lipid based dispersion as described in anyone of embodiments 1-42 wherein the therapeutic agent is an anesthetic or a sedative.
48. The method of embodiment 47 wherein the therapeutic agent is propofol.
49. A method for producing an antineoplastic effect in an animal comprising administering to the animal an effective amount of a lipid based dispersion as described in anyone of embodiments 1-42 wherein the therapeutic agent an antineoplastic agent.
50. The method of embodiment 49 wherein the antineoplastic agent is etoposide.
51. The method of embodiment 49 wherein the antineoplastic agent is paclitaxel.
52. A method for producing an immunosuppressive effect in an animal comprising administering to the animal an effective amount of a lipid based dispersion as described in anyone of embodiments 1-42 wherein the therapeutic agent is an immunosuppressive agent.
53. The method of embodiment 52 wherein the immunosuppressive agent is cyclosporine.
54. A method for treating atherosclerosis, atherosclerotic vulnerable plaque or restenosis, or a combination thereof, in an animal, comprising administering to the animal an effective amount of a lipid based dispersion as described in any one of embodiments 1-42 wherein the therapeutic agent is an photoreactive agent.
55. The method of embodiment 54, wherein the photoreactive agent is gallium deuteroporphyrin dimethyl ester.
56. The method of embodiment 54 wherein the photoreactive agent is gallium deuteroporphyrin dimethyl ester and wherein the lipid dispersion comprises Soy PC and DSPG in a mole ratio of 1:0.1 to 1:0.4 Soy PC:DSPG.
57. A lipid-based dispersion as described in anyone of embodiments 1-42 for use in medical therapy.
58. The use of a lipid based dispersion as described in anyone of embodiments 1-32 and 34-42 wherein the therapeutic agent is an anesthetic or a sedative to prepare a medicament useful for producing an anesthetic or sedative effect in a mammal.
59. The use of embodiment 58 wherein the therapeutic agent is propofol.
60. The use of a lipid based dispersion as described in anyone of embodiments 1-32 and 34-42 wherein the therapeutic agent an antineoplastic agent, to prepare a medicament useful for producing an antineoplastic effect in a mammal.
61. The use of embodiment 60 wherein the antineoplastic agent is etoposide.
62. The use of embodiment 60 wherein the antineoplastic agent is paclitaxel.
63. The use of a lipid based dispersion as described in anyone of embodiments 1-32 and 34-42 wherein the therapeutic agent is an immunosuppressant to prepare a medicament useful for producing an immunosuppressant effect in a mammal.
64. The use of embodiment 63 wherein the immunosuppressant is cyclosporine.

## Claims

1. A lipid-based dispersion comprising, a) phosphatidyl choline; b) an anionic phospholipid; optionally c) up to 1% cholesterol by weight of total lipids; and optionally d) a therapeutic agent, wherein the mean particle size measured by dynamic light scattering is less than 100 nm.

2. The lipid-based dispersion of claim 1 wherein at least about 60% of the fatty-acid chains of the phosphatidyl choline comprise 16 or more carbon atoms or 18 or more carbon atoms.

3. The lipid-based dispersion of claim 1 or 2 wherein at least 50% of the fatty-acid chains of the phosphatidyl choline comprise at least one double bond.

4. The lipid-based dispersion of claim 1 wherein the phosphatidyl choline is selected from Soy-PC, Egg-PC, DEPC, and DOPC.

5. The lipid-based dispersion of any one of claims 1-4 that comprises less than 0.5% cholesterol.

6. The lipid-based dispersion of any one of claims 1-5 wherein at least about 60% of the fatty-acid chains of the anionic phosphohpid comprise 16 or more carbon atoms or 18 or more carbon atoms.

7. The lipid-based dispersion of any one of claims 1-5 wherein the anionic phospholipid is selected from Egg-PG, Soy-PG, DSPG, DPPG, DEPG, DOPG, DSPA, DPPA, DEPA, DOPA, DSPS, DPPS, DEPS, and DOPS, and mixtures thereof.

8. The lipid-based dispersion of any one of claims 1-5 wherein the anionic phospholipid is DSPG.

9. The lipid-based dispersion of any one of claims 1-8 wherein the therapeutic agent is etoposide, propofol, cyclosporin, or paclitaxel.

10. The lipid-based dispersion of any one of claims 1-9 that comprises liposomes.

11. The lipid-based dispersion of claim 10 wherein the liposomes have a melting temperature below 35°C

12. The lipid-based dispersion of any one of claims 1-11 which comprises from 0.05 to 60 % anionic phospholipid by mole relative to phosphatidyl choline.

13. The lipid-based dispersion of any one of claims 1-12 wherein the weight ratio of total lipid (phosphatidyl choline + anionic phospholipid) to therapeutic agent is greater than 1.1.

14. A unit dosage form comprising a lipid-based dispersion of any one of claims 1-13.

15. A method for modulating the solubility of a therapeutic agent comprising incorporating the agent in a lipid-based dispersion as described in any one of claims 1-13.

16. A lipid based dispersion as described in any one of claims 1-13 wherein the therapeutic agent is an anesthetic or a sedative for use in a method of producing an anesthetic or sedative effect in a mammal, for producing an antineoplastic effect in a mammal or for producing an immunosuppressant effect in a mammal.

17. A lipid based dispersion as described in any one of claims 1-13 wherein the therapeutic agent is an photoreactive agent for use in a method of treating atherosclerosis, atherosclerotic vulnerable plaque or restenosis, or a combination thereof, in an animal.
